# EUROPEAN PATENT APPLICATION

(11) **EP 4 012 717 A1**
(43) Date of publication of application: **15.06.2022**
(21) Application number: 20212416.0
(22) Date of filing: 08.12.2020
(51) Int. Cl.: G16H 10/60, G16H 50/20, G16H 50/30, G16H 50/70, G16H 70/60, A61B 5/00, G16H 15/00, G16H 20/00, G16H 80/00, G16H 10/40, G16H 10/20

(54) **A PREGNANCY DECISION SUPPORT SYSTEM AND METHOD**

(71) Applicant: Koninklijke Philips N.V., 5656 AG Eindhoven (NL)
(72) Inventor: HAMELMANN, Paul Christoph, 5656 AE Eindhoven (NL); LONG, Xi, 5656 AE Eindhoven (NL); ASVADI, Sima, 5656 AE Eindhoven (NL); RABOTTI, Chiara, 5656 AE Eindhoven (NL); SIMONS, Evelyn Josefina Maria, 5656 AE Eindhoven (NL); VAN BENTUM, Jesper William, 5656 AE Eindhoven (NL); VAN SAMBEEK, Shannon, 5656 AE Eindhoven (NL); VAN DER HOUT, Beatrijs, 5656 AE Eindhoven (NL)
(74) Representative: Philips Intellectual Property & Standards

(57) **Abstract**

A pregnancy decision support system is for identifying potentially high-risk combinations of pregnancy-related symptoms. A categorization of symptoms, provided by a health care professional, is received that a pregnant woman may report. A model is then built for identifying potentially high-risk combinations of pregnancy-related symptoms based on the received categorization of symptoms. Symptoms are reported by the pregnant woman and they are applied to model to determine a proposed course of action. This provides an automated analysis of symptoms and supports clinical decision making.

## Description

### FIELD OF THE INVENTION

The invention relates to a system and method for providing support to an expectant mother and the health care provider (e.g. obstetric gynecologist) during pregnancy, in particular to provide support in making clinical decisions in response to symptoms, which have been experienced by the pregnant woman.

### BACKGROUND OF THE INVENTION

Various societal and population health trends, such as advanced maternal age at the time of first pregnancy, increasing incidence of high BMI and chronic stress as well as chronic conditions such as diabetes and hypertension, are contributing factors in creating increasingly complicated pregnancies.

Globally, on average 10% (ranging from 3-18% depending on the study population) of pregnancies are complicated with hypertensive disorders of pregnancy (HDP) including pre-eclampsia. In recent decades there has been a progressive increase in the prevalence of HDP both in the US and Europe.

HDP (including preeclampsia) is a key pregnancy complication, in which early identification is important to prevent and/or delay the onset of the condition and/or conduct effective monitoring of high-risk cases. Any potential benefit that behavioral interventions might have in preventing pregnancy risks will be effective if initiated in early pregnancy. In current practice however, intervention is initiated only after the condition has been diagnosed, which is typically later in pregnancy, when it is too late for prevention of both the conditions during pregnancy and their adverse consequences throughout the life of the mother and the new born.

Pregnancy is generally a monitored process. This is partly because apparent maternal (and paternal) health does not necessarily guarantee a completely risk free maternal and fetal outcome. Pregnancy monitoring usually starts in the first trimester, soon after pregnancy is confirmed, and is continued until delivery in regulated intervals. If certain initial risk factors (such as blood pressure above a certain threshold; typically above 140 mmHg systolic blood pressure and 90 mmHg diastolic blood pressure) are present during any of the routine checks the monitoring process will also include the follow up of the known symptoms based on which health care professionals conduct further tests. These tests include kidney function biomarkers, namely urine protein level, serum Creatinine and uric acid. In the case of more severe preeclampsia, enzymes that indicate liver function and platelet levels are among the biomarkers that can be monitored. Interventions are prescribed based on the combined outcome of the monitoring of symptoms and if necessary testing. This process requires frequent routine checks for which pregnant women have to travel to the hospital or are even hospitalized. It is generally difficult to combine all determining factors for multiple reasons outlined below.

Pregnancy complications are often accompanied by symptoms. However, apart from the presence of the symptoms, multiple additional factors such as their severity, duration and rate of progression has to be taken into account. Such evaluation of symptoms requires significant experience and it is doubly challenging as the health care professional needs to determine the parameters by asking the relevant questions and subjectively combine all factors during the short period of time of the routine antenatal checks.

The reporting of symptoms is subjective, and it is difficult for patients to remember all symptoms, hence, evaluation may be inaccurate.

Even normal pregnancies cause various discomforts and symptoms. It is often difficult to distinguish a normal pregnancy from a complicated one based on discomforts and symptoms.

Finally, single symptoms may not be indicative of any risks depending on their severity and persistence, but a combination and/or aggregation of symptoms even when mild or not continuously present may be warning signals of events to come.

There are various known systems for general symptom checking. The majority of consumer facing symptom checkers are solutions with the primary function to facilitate self-diagnosis. Some others are designed to assist patients with a life-threatening problem, such as a stroke or heart attack, to seek emergency care. Such products also aim to offer reassurance to patients with non-urgent problems, and recommend self-care techniques.

Among consumer facing apps, Babylon Health (Trade Mark) is one example of the leading companies. However, diagnosis is sometimes incorrect, and it has for example been demonstrated that doctors make a correct diagnosis, on average, more than twice as often as online symptom-checkers.

Apart from these consumer-facing apps, certain providers offer symptom checkers on their patient portals and websites. The algorithms used vary across each solution, ranging from branching logic, Bayesian inference, or other proprietary methods, but they all invariably rely on models for combining the symptoms into a diagnosis, which are predefined and static, making the diagnosis accuracy inferior to diagnosis of a healthcare practitioner.

These general and broad symptom checkers are too non-specific for pregnancy.

The article "Simple Screening for High-Risk Pregnancies in Rural Areas Based on an Expert System" of R. Supriyanti et. al., Telkomnika, Vol. 13, No. 2, June 2015 pp. 661-669 discloses the use of the Analytical Hierarchy Process to make decisions about potentially high risk pregnancy patients.

### SUMMARY OF THE INVENTION

The invention is defined by the claims.

According to examples in accordance with an aspect of the invention, there is provided a decision support system for identifying potentially high-risk combinations of pregnancy-related symptoms, comprising:
a first input interface for receiving a categorization of symptoms that a pregnant woman may report;
a model-building unit adapted to build a model for identifying potentially high-risk combinations of pregnancy-related symptoms based on the received categorization of symptoms;
a second input interface for receiving information about symptoms reported by the pregnant woman;
a controller adapted to apply the reported symptoms to the model and thereby determine a proposed course of action; and
an output interface for outputting the proposed course of action.

This system creates a model that is specific to one or more major pregnancy complications for which the accepted clinical diagnosis is based on symptom tracking and monitoring. The system performs grading (i.e. creating different weightings) based on received symptom categorizations and aggregation. The system can be customized based on the categorizations that are provided to it, for example it can be based on, and integrated with, a practice protocol of a hospital and/or clinic, a groups of specialists, or even preferences of a single healthcare professional. Hence, it can accommodate the development of the professionals' expertise, and the model can be updated to take account of these developments. These developments can be integrated into the basic model for a reliable diagnosis and triage.

The invention is of particular interest for conditions for which there is not a single diagnostic test for diagnosis, but where the symptom assessment is a critical part of the diagnosis.

The system may also take into account the evolution of symptoms over time, by applying reported symptoms over time to the model, and not only reporting instantaneous symptoms.

The first input interface may be further for receiving a categorization of symptom attributes comprising one or more of duration, frequency, intensity and location. Thus, the type of symptom as well as the more specific nature of the symptoms can be taken into account in the generated weightings.

The first input interface may be for receiving different categorizations of symptom attributes for different phases of pregnancy. Thus, the proposed course of action may depend on the stage of pregnancy.

The proposed course of action for example indicates whether the pregnant women should seek medical assistance and/or indicates a type of care package that should be implemented by a medical professional.

For providing proposals for action by the pregnant woman, the proposed course of action is for example selected from a list which includes at least:
do not seek medical assistance; and
seek medical assistance.

Thus, the system provides a simple output for a user, to reassure them or to advise seeking medical assistance. There may be other courses of action, such as monitoring symptoms more regularly over a next time period.

There may also be proposed actions for a medical professional, such as:
assign a high-risk care path to patient;
schedule more antenatal care visits;
order additional tests for definitive diagnosis;
communicate preventive measures to the patient;
adjust (e.g. pharmacological) intervention;
communicate suggested behavioral changes.

The model-building unit may be adapted to apply an analytic hierarchy process (AHP). This is one possible way to implement the model building, to provide weightings of different symptoms (and attributes). Other analytical multi-criteria decision modelling methods may be used, such as aggregated indices randomization method (AIRM), analytic network process (ANP), multi-attribute global inference of quality (MAGIQ), weighted sum model (WSM), best worst method (BWM), and decision expert (DEX).

In addition to these, semi-supervised or unsupervised machine learning (or artificial intelligence) methods may be used.

The model-building unit may for example be adapted to generate a pairwise comparison matrix of symptoms and calculate therefrom weights of each symptom. This applies to certain methods, such as AHP, that require performing pairwise comparison.

The symptoms for example comprise a plurality selected from: headache; edema; visual disturbances; abdominal pain; pain in the stomach pit; pain between the shoulders; nausea; vomiting; loss of appetite; malaise; loss of fetal motion; finger tingling; urinary problems; hyper sensitivity; and hyperreflexia. There may of course be other symptoms that can be added to this list.

These are symptoms commonly experienced during pregnancy, including symptoms that may be routine and harmless, but wherein different combinations of symptoms may be indicative of particular medical conditions.

For different complications for a subject, other symptoms are important.

Other risk factors may also be combined into the model, such as medical history, age, blood pressure etc.

The invention also provides a computer implemented method for identifying potentially high-risk combinations of pregnancy-related symptoms, comprising:
receiving a categorization of symptoms that a pregnant woman may report;
building a model for identifying potentially high-risk combinations of pregnancy-related symptoms based on the received categorization of symptoms;
receiving information about symptoms reported by the pregnant woman;
applying the reported symptoms to the model and thereby determining a proposed course of action; and
outputting the proposed course of action.

This is the method implemented by the system defined above.

The method may comprise receiving the categorization of symptoms from multiple medical professionals as qualitative data, and the method comprises converting the qualitative data into quantitative data before building the model. This simplifies the input of data to the system for the medical professionals.

The method may comprise building a first model based on received categorizations from multiple medical professionals and building a second model based on received categorization of symptoms from an individual medical professional. This enables an individual medical professional to compare their judgements with those formed from a panel of experts.

The method may comprise building a first model based on received categorizations from a first group of multiple medical professionals and building a second model based on received categorization of symptoms from a second group of multiple medical professionals. This enables a user to compare judgements of different groups of medical professionals, e.g. midwives and doctors.

There may be more than two models, for example for an individual user and for multiple different groups.

The method may comprise receiving a categorization of symptoms derived from medical professional surveys or from a user interface allowing the categorizations to be applied to the symptoms.

The method may comprise receiving a categorization of symptom attributes comprising one or more of duration, frequency, intensity and location. Different categorizations of symptom attributes may be received for different phases of pregnancy.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a pregnancy decision support system;
Figure 2 shows an example of constructing a symptom aggregation model with three layers using AHP;
Figure 3 shows a list of symptoms and associated system attributes and categorizations in the form of rankings applied to them;
Figure 4 shows a table of scores for three symptoms for the first layer of the hierarchy;
Figure 5 shows an example of the resulting weights for the three symptoms as well as their attributes;
Figure 6 shows an example calculation of the weights;
Figures 7 to 12 show three examples to demonstrate how the output of the model depends on the presence of symptoms;
Figure 13 shows an example of an elevated risk over a period of 6 days;
Figure 14 shows how the model may be extended by including context information;
Figure 15 shows the outcome of the symptom aggregation model compared between a group and a customized model;
Figure 16 shows that the differences of Figure 15 may also be broken down into the different symptoms;
Figure 17 shows the outcome of the model for gynecologists vs. nurses vs. midwives; and
Figure 18 shows that the symptom attributes may be collected from the user via a Smartphone app.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a clinical decision support system for identifying potentially high-risk combinations of pregnancy-related symptoms. A categorization, such as a ranking, comparison or clustering of symptoms, provided by a health care professional, is received that a pregnant woman may report. A model is then built for identifying potentially high-risk combinations of pregnancy-related symptoms based on the received categorization of symptoms. Symptoms are reported by the pregnant woman and they are applied to model to determine and facilitate taking the appropriate course of action. This provides an automated analysis of symptoms.

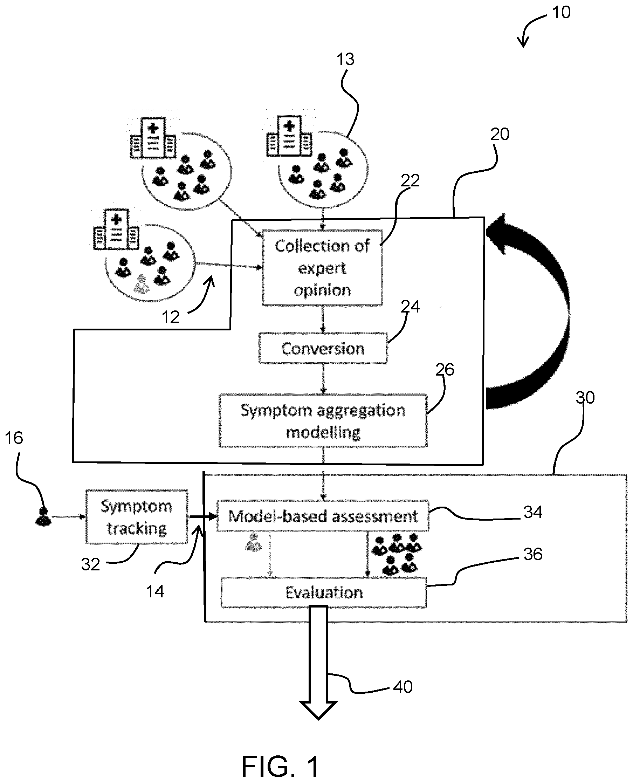

Figure 1 shows the pregnancy decision support system 10. There is a first input interface 12 for receiving a categorization of symptoms that a pregnant woman may report. These reports from example are provided by groups medical professionals 13.

A second input interface 14 is for receiving information about symptoms reported by a pregnant woman 16.

A model-building unit 20 builds a model for identifying potentially high-risk combinations of pregnancy-related symptoms based on the received categorization of symptoms. The model-building unit 20 has a first unit 22 for collection of the expert opinions from the medical professionals 13. The categorization of symptoms is for example qualitative data, and a conversion unit 24 converts the qualitative data into quantitative data before building the model.

The building of the model, based on the quantitative data, takes place in the symptom aggregation modeling unit 26.

A controller 30 applies the reported symptoms received at the second input interface 14 to the model in a model-based assessment unit 34 and thereby determines a proposed course of action in an evaluation unit 36. An output interface 40 outputs the proposed course of action.

The symptoms of the pregnant woman 16 (i.e. the patient) may be provided directly by the woman and/or by a symptom tracking unit 32 comprising physiological sensors.

The system may generate multiple models, from different categorizations of symptoms. For example, a first model may be based on a large number of medical professionals, and an individual medical professional may create their own model based on their own categorizations. Such an individual model may fits the individual preferences better (e.g. because it fits better in the clinical workflow).

The model-building unit 20 is a first part for model creation and the controller 30 is a second part for model deployment.

In the first part, the opinion of multiple experts on the importance of symptoms for their assessment is collected. This can, for example, be done via surveys and/or an online dashboard. This qualitative symptom data is converted into quantitative symptom data used for the symptom aggregation model. Over time, more expert opinions may be collected or experts may want to adjust or re-customize their model.

In the second part, the symptoms of the patient are tracked and evaluated using the model created in the first part.

A healthcare professional may compare the outcome provided by a model based on his own categorizations to a model based on the categorizations derived of the multiple experts. In this way, the health care professional is able to make an informed and conscious decision.

The (or each) built model may be a sequence-model, a mixed-model, an extended-model, and/or a customized model.

The symptoms to be monitored may comprise symptom types as well as symptom attributes (hence multi-dimensional data) such as duration, frequency, intensity/severity, location, etc. This data may all be used as criteria to build the symptom aggregation models. These attributes are possibly collected in different phases of the pregnancy (e.g. trimesters), which means that symptom aggregation models can be specific in those different phases.

An example will now be provided of a basic multi-criteria decision making method using an analytic hierarchy process (AHP) as described in T. L. Saaty, "The analytic network process", Int. Ser. Oper. Res. Manag. Sci., vol. 95, pp. 1-26, 2006.

The AHP method is based on a pairwise comparison between all criteria (including the symptoms and the symptom attributes). In that way, a user when inputting weighting information only has to compare two criteria at a time with respect to a goal, which makes the assessment of criteria much easier.

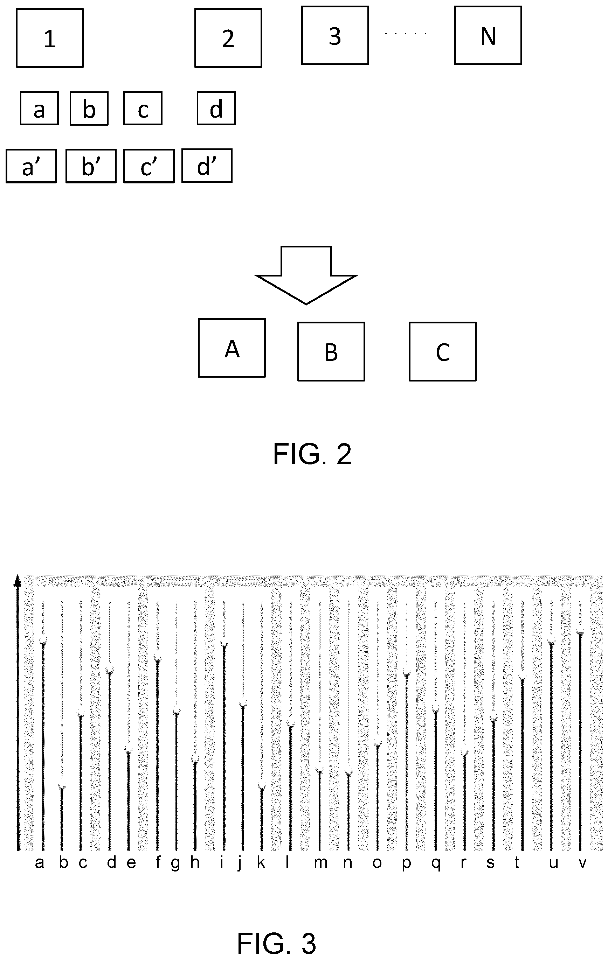

Figure 2 shows an example of constructing a symptom aggregation model with three layer using AHP. The goal is to decide which action to take, including three alternatives: A do nothing, B call the midwife, and C take rest and keep track. In other examples, there may be different goals, such as to decide which care path a pregnant women should be allocated to. A: low risk (normal) care path, B: high risk care path (with more visits).

The first layer is the symptoms 1 to N.

The symptoms are for example selected from Edema, Headache, Vision impairment, Abdominal pain, Sweating, Pain in the pit of the stomach, Nausea, No appetite, Vomiting, Malaise, Can't feel the baby, Pain between shoulder blades, Tingling, Urine frequency, Hyper sensitivity and Hyperreflexia.

Some but not necessarily all of these symptoms have attributes a to d in the second layer. For example, a headache may be report in the front, back or side. Abdominal pain may be reported in the upper, lower or side of the abdomen. Edema may be reported in the face, feet or fingers.

Some but not necessarily all of these symptoms have attributes a' to d' in the third layer, for example the intensity, frequency, regularity or duration of the symptom.

Given a final goal (i.e. outcomes A, B or C) AHP is able to build a group (or collaborative) decision-making model based on the results of questionnaire(s) provided by clinicians. The modelling step aims to train the model with pre-determined weights of factors (i.e. symptom attributes) by incorporating and harmonizing inputs from multiple clinicians to reach consensus.

The pairwise comparisons are able to produce an accurate quantification of the weights of the symptom attributes in multiple layers, by comparing the relative importance between each two symptom attributes in one layer using a specific questionnaire. Typical questions can be a 9-point scale in terms of attribute or factor importance:
1. Equal importance
2. Weak importance of one over another
3. Moderate importance of one over another
4. Moderate plus importance of one over another
5. Strong importance of one over another
6. Strong plus importance of one over another
7. Very strong importance of one over another
8. Very, very strong importance of one over another
9. Extreme importance of one over another

Other scales are of course also possible such as the 5-, 7-, or 10-point Likert scale or a binary Yes/No response.

A final goal is set for the multiple-criteria decision analysis. For example, the goal can be to assess whether a pregnant woman has an increased risk of developing HDP, or which of the actions A to C she needs to take.

In the following, details are provided for a simplified example. Here, only three symptoms are considered and the goal is choose between only two alternatives (alternative A of a low symptom aggregation profile i.e. low risk for a particular condition e.g. HDP and alternative B of a high symptom aggregation profile i.e. high risk) for making a decision. The goal is then to determine the symptom aggregation profile of the patient.

In addition, the opinion of only a single health care professional is considered. A healthcare professional is asked to do a pairwise comparison of the criteria under consideration. This is for example in the form of a ranking between those pairs.

In one solution, this may be implemented via an online questionnaire. For example, "How much more important do you think is symptom A with respect to B". Alternatively, a healthcare professional may be asked to use a dashboard such as illustrated in Figure 3.

Figure 3 shows a list of symptoms and associated system attributes, a to v.

For example, these symptoms and attributes are:
a. Headache front
b. Headache back
c. Headache side
d. Flashing sight
e. Blurred vision
f. Abdominal pain at the top
g. Abdominal pain on the side
h. Abdominal paid at the bottom
i. Edema in the face
j. Edema in the hands
k. Edema in the feet
l Pain in the pit of the stomach
m. Nausea
n. No appetite
o. Vomiting
p. Malaise
q. Can't feel the baby
r. Pain between shoulder blades
s. Tingling
t. Urine problems
u. Hyper sensitive
v. Hyperreflexia

The healthcare profession can move sliders for each symptom to express their perception of the relative importance of each, and thereby provide a categorization of each symptom.

Other approaches to obtain the clinician's opinion are possible.

The input by the clinician is used to create a pairwise comparison matrix for each node in the hierarchy, and used to calculate the priorities (weights) of each symptom relevant for the decision making process.

For this example, the three symptoms to be assessed are headache, edema and malaise.

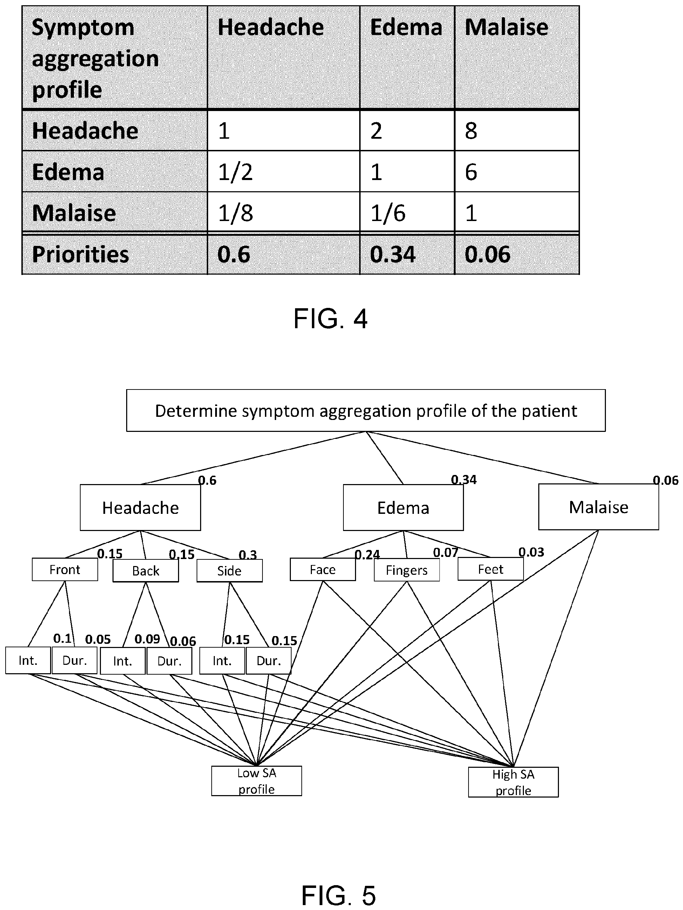

Figure 4 shows a table of scores for these three symptoms for the first layer of the hierarchy (i.e. the basic symptom with no attribute).

This table shows an example of the input given by a clinician for that first layer of the hierarchy. The clinician compares the symptoms in pairs with respect to the goal of selecting the correct symptom aggregation profile. In this example, Headache is weakly more important (i.e. value 2) than Edema but has very strong importance (i.e. value 8) compared to Malaise. The priorities (weights) are calculated by raising the matrix to large powers and summing each row and dividing each by the total sum of all the rows, as described by "The analytic network process" of Saaty, referenced above.

This process is repeated for each node in the hierarchy (hence all symptoms and all attributes) to compute all weights in the structure.

Figure 5 shows an example of the resulting weights for the three symptoms as well as their attributes. The goal is to determine the symptom aggregation profile (high or low) of a patient. The calculated importance of the symptoms and attributes are written next to each node.

This assessment may only be a one-time action but may be repeated if a clinician wants to update or modify their input and corresponding opinion. When the clinician has done all pairwise comparisons, the symptom aggregation model is complete, i.e. all the weights can be calculated. The clinician could also repeat this action to learn whether his assessment leads to the same weights. If there are discrepancies, the average weight could be taken to generate a more accurate model.

The input of the clinician leads to a model with weights defining how to what extent each criteria contributes to the decision making process, i.e. determining the correct symptom aggregation profile of the patient. Whether the low symptom aggregation profile or the high symptom aggregation profile is the correct choice depends on the actual presence of the symptoms reported. If no symptoms are reported, both symptom aggregation profiles will have equal weight of 0.5.

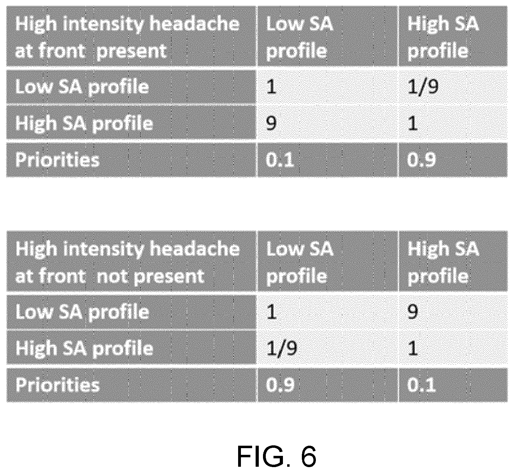

Figure 6 shows an example calculation of the weights when a high intensity headache is present using again the approach of pairwise comparison.

For a high intensity headache at the front of the head (the top table), a high symptom aggregation profile is a far more appropriate alternative compared to the low symptom aggregation profile.

Likewise, when there is no high intensity headache at the front of the head (the bottom table), a low symptom aggregation profile is a far more appropriate alternative compared to the high symptom aggregation profile.

Note that the pairwise comparison matrix in Figure 6 is predefined and used for the automatic calculation of the alternative weights.

If attributes such as intensities are not tracked in this binary way but using a continuous scale, the pairwise comparison matrix can be adjusted accordingly.

Eventually, the weights for the alternatives (i.e. the low symptom aggregation and high symptom aggregation profile) are summed for all the lowest level nodes in the hierarchy to compute the aggregated weights for the alternatives.

In the above example, the weights for the alternatives are automatically calculated based on the presence (or attribute value) of the reported symptoms. However, a clinician may also want to customize the weights in this lowest level of the hierarchy.

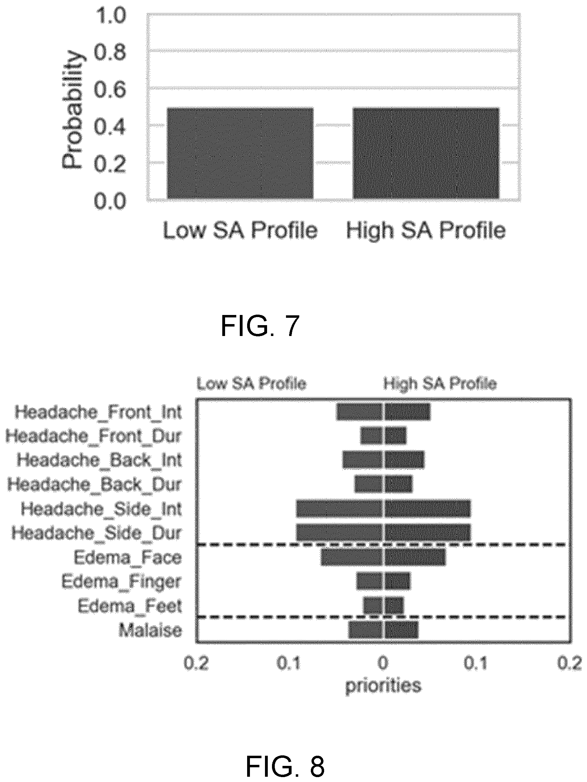

Figures 7 to 12 show three examples to demonstrate how the output of the model depends on the presence of symptoms. In Figure 7 an example is given in which no symptoms are reported. Logically, both alternatives are equally good choices for the goal. The priorities of the individual symptom attributes are shown in Figure 8.

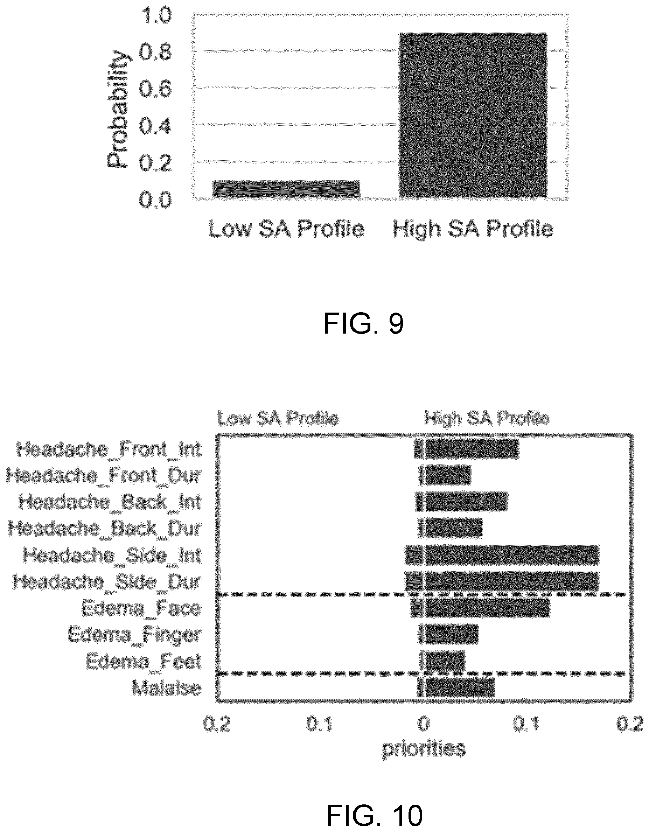

In Figure 9 an example is shown in which all symptoms are present, such that the high symptom aggregation profile is the best choice. Figure 10 shows the corresponding individual priorities.

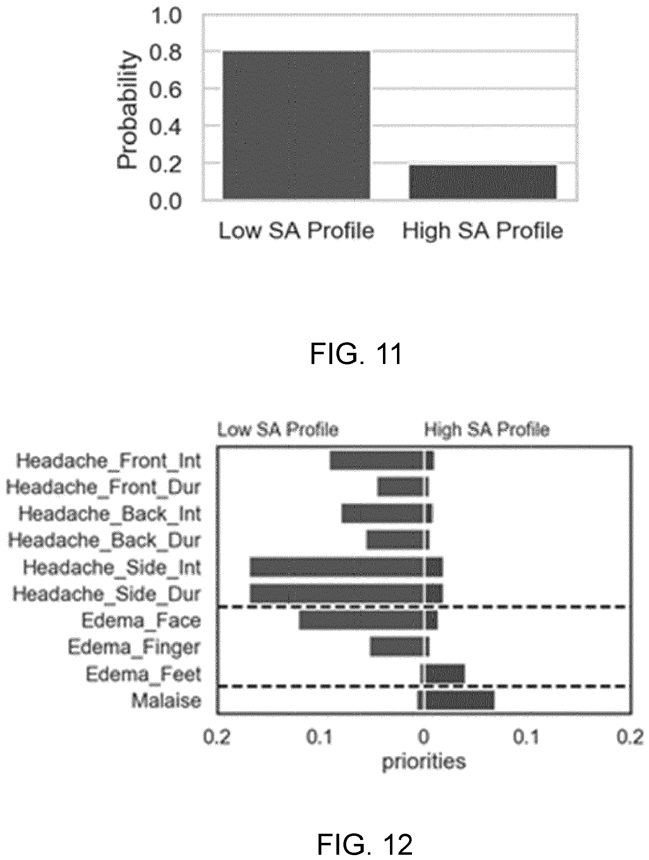

In Figure 11 an example is shown in which most of the symptoms are not present and symptoms with lower weights (Edema in Feet, Malaise) are present. The model says that the low symptom aggregation profile is the best choice. Figure 12 shows the corresponding individual priorities.

In addition, historical symptom attributes can be taken into account over time in the modelling (i.e. the sequences of symptom attributes), which enables time-dependent dynamic symptom aggregations. Symptoms are collected on a regular base (e.g. daily or weekly), where all symptoms in the preceding days (e.g. three days) or weeks (e.g. 3 weeks) are used to determine whether a pregnant woman is in the low or high symptom aggregation profile. In this case, the model creation should can take into account the assessment of the symptom importance of previous days or weeks.

When collecting symptoms, a specific order can be applied and questions can be simplified to make decisions. For example, if there is intensive and frequent abdominal pain, whether edema occurs and how intensive it is should be asked, and then whether the blood pressure is high; and if yes, high symptom aggregation should be the case.

Alternatively, a sequence of symptoms can be considered as an additional attribute. For example, when there are consecutive days of headache, this time-dependent criterion should have a higher importance then the headache happening only during one of those days.

In addition, by fitting estimated probabilities over time, an overview is available of the time-varying high/low risk probability. This allows clinicians to examine the trend of the risk and see whether it is increasing, which can improve decision-making. If the fitted trend (slope) is larger than a threshold (e.g. >0.04) and the probability of the latest high symptom aggregation risk is larger than a threshold (e.g. > 0.4), the system can set a flag.

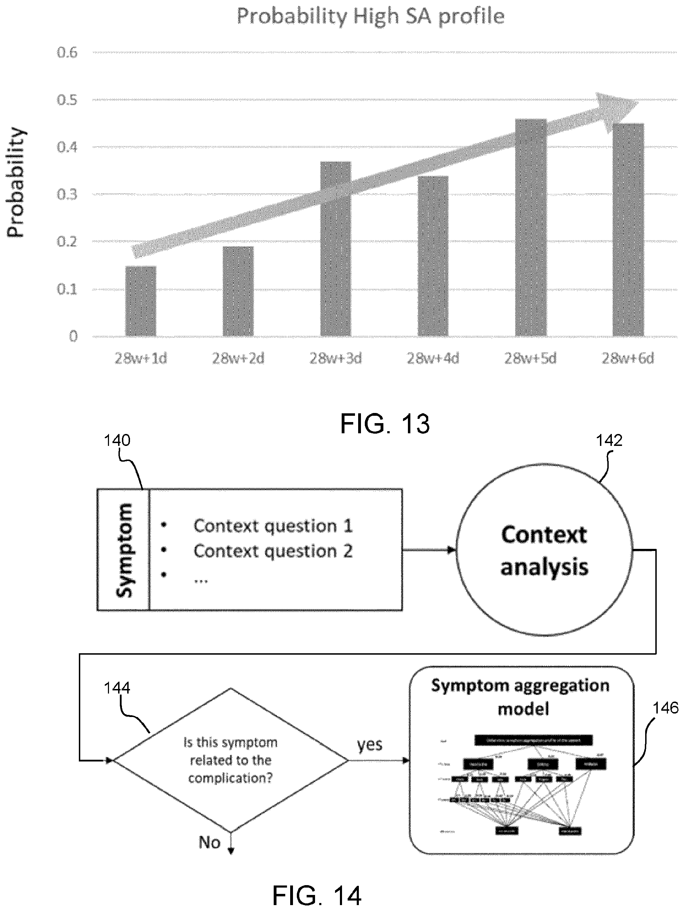

Figure 13 shows an example of an elevated risk over a period of 6 days. Although the probability of having a high symptom aggregation risk profile (the y-axis) is still lower than 50%, attention should be paid to this pregnant woman.

When building the symptom aggregation model, opinions from clinicians (results of a questionnaire, interviews, or dashboards (as described above)) are required as input. However, the occurrence of symptoms also depends on daily-life context. It is known that context is very important when receiving patients' symptoms. Context will point out which symptoms relates to the complication (e.g., HDP) and which do not. For example, a headache is a likely outcome of dehydration (by sitting in the sun and not drinking enough water). A headache is also a likely result of a stressful day. Therefore, extending the modelling by considering the context is of interest.

Figure 14 shows how the model may be extended by adding a step 140 of receiving context information as part of the symptom reporting and in step 142 analyzing the context information for the symptom aggregation model. In step 144, it is determined if the symptom is related to the complication (i.e. medical condition being assessed). If not, because the symptom relates to the context, then the model is not used. Otherwise, the model 146 is used.

In this way, important context information highly influencing symptoms can be pre-defined by the clinicians and then collected by asking additional questions to mothers. After that, the AHP symptom aggregation model takes into account this context information by filtering out symptoms unrelated to the targeted complication.

When collecting large amounts of data, how the context information influences the AHP decision-making and clinical outcome can be learned. A machine-learning algorithm can be trained based on these data and further help clinicians to make decisions with an increased adequacy.

It is mentioned above that the opinion of a single expert can be quantified and used for model creation. By obtaining the input of multiple experts, a more accurate more generalizable model can be created. However, it should be noted that the modelling approach may not require intensive data collection because a model based on the input of a single health care professional is already clinically valuable.

The modelling approach offers the opportunity for customization. For example, an individual health care professional may have good reasons to believe that a certain symptom should receive a higher weight, e.g. based on new clinical insights or based on the availability of resources in his health institute. The health care professional can then generate the model and evaluate the model outcome against the opinion of the group of experts. As the model provides the individual priorities generated by the group, different risk probabilities can easily be understood and taken into account when making a final decision.

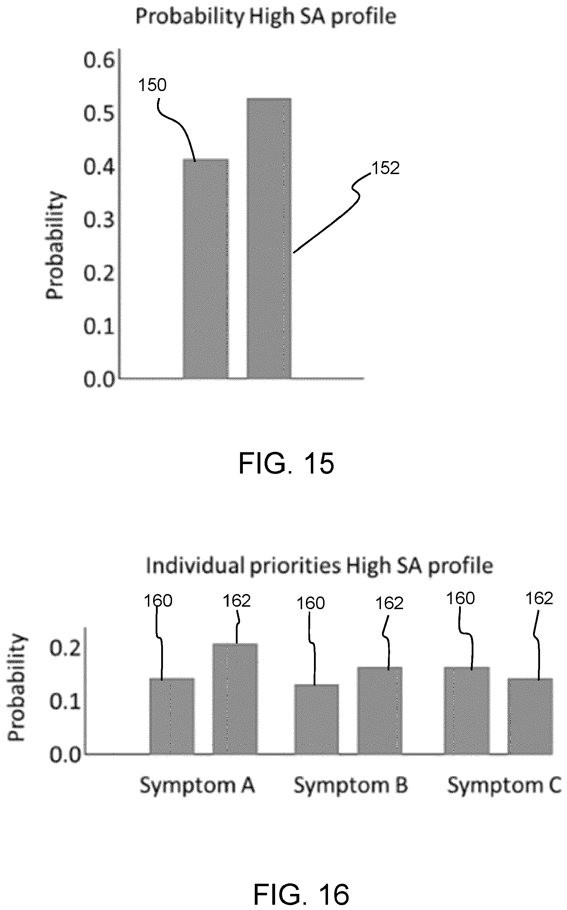

For example, Figure 15 shows the outcome of the symptom aggregation model (as a probability of the patient being in the high symptom aggregation profile) compared between the group (bar 150) and a customized model (bar 152).

Figure 16 shows that these differences may also be broken down into the different symptoms, wherein the left bar 160 in each case is the group result and the right bar 162 is the customized result.

In addition, the modelling approach allows insights to be obtained into the symptom relevance as seen by different groups of health care professionals.

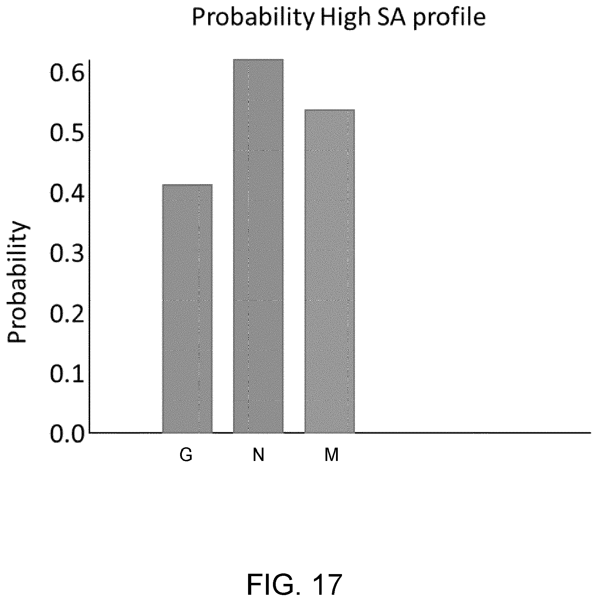

Figure 17 shows the outcome of the model for gynecologists G vs. nurses N vs. midwives M. Likewise, the outcome of a model created by a different group of clinicians in the same hospital, created by a different hospitals, created in a different regions or countries or societies, or created on a different ethnicity, can be compared. This enables learning from different specialties and promotes the sharing of clinical insights.

When the system is used over a long period, a large set of symptom-data is collected. Including the eventual diagnosis given by the clinician, this collected data can help to create symptom-based predictive models, e.g. using different machine learning algorithms. In that way, the clinical expert's opinion is validated or new symptom insights can be created. Furthermore, it allows providing information to the clinician on how many patients with a specific set of symptoms were diagnosed with a certain complication.

The system may be used to help the user, i.e. a pregnant woman or healthcare professionals, to make decisions by collecting symptom attributes. After finishing a user questionnaire indicating the occurrence of certain symptoms and other attributes of the symptoms (duration, frequency, intensity) or reporting symptoms, the answers can be fed into the pre-designed trained model.

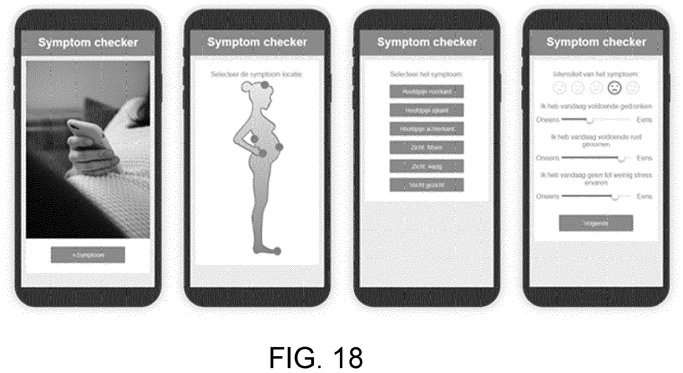

Figure 18 shows that the symptom attributes may be collected from the user via a Smartphone app. Alternatively, via web-based user dashboards may be used. By collecting the symptom attributes (severity, duration etc.), the lowest level in the AHP network, i.e. the pairwise comparison matrices for the alternatives, are defined.

The tracked and aggregated symptoms can be used by the user (health care professional or pregnant mother) to make a decision. For example, a pregnant mother could get a notification whether she should or should not call the midwifery practice for further support. At the same time, the symptom data and calculated symptom-based risk is shared to the health care professional. For example, the symptom data and calculated symptom-based risk may be directly integrated into the electronic medical record.

In another example, the tracked symptoms may be visualized in a dashboard for the healthcare professional. The health care professional can see the occurrence of symptoms as a function of time and a corresponding symptom aggregation risk flags. The health care professional may click on a button to obtain more information about the outcome of the model, i.e. the relevance of the individual symptoms for the decision making process (for example a similar visualization as shown in Figures 7 to 12). In this way, the outcome of the model is fully transparent where the factor weights determined during modelling and the final decision path are visible, accountable and interpretable. This is more acceptable to healthcare professionals and users in healthcare applications compared to a 'black-box' model.

The dashboard may contain more clinical relevant information such as lab-results (e.g. blood pressure or protein in urine). Also contextual information may be tracked/included to correct the symptom relevance based on behavioral or environmental influences, as explained above.

The system may thus be used to empower patients and care providers in the prenatal care journey to improve the experience and efficiency and ultimately health outcomes. With this system, both expectant mothers as well as care providers can be supported by creating awareness of changing health indicators, tracking relevant parameters in a structured way and sharing data between the expectant mother and the care provider. Potential risks can be detected earlier.

The invention provides a system which uses a dedicated list of symptoms for monitoring or tracking throughout pregnancy. Trimester-specific models are preferably generated, because the symptom relevance changes over time.

The symptoms are classified by type but preferably also by other criteria or features such as frequency, intensity and duration. These criteria are important for the clinical decision making.

Customized models may be provided giving insights into the relevance of symptoms as regarded by different healthcare professionals, i.e. midwives vs. gynecologists. The models may indicate how much each individual symptom contributes to the decision making process based on the input of different users. This allows for transparent visualization, comparison and ranking of symptom importance.

The invention may be used for continuous monitoring of symptoms, which may be used in sequential models for clinical decision support. The outcome of the model may be compared against contextual information provided by the user.

In the above detailed description above, a symptom aggregation model for hypertensive disorder (HDP) is given as an example, but the invention can be applied to other conditions during pregnancy, such as hypoglycemia in pregnancy or hyperglycemia in pregnancy.

The analytic hierarchy process (AHP) methods are described in detail for the symptom aggregation modelling, but the invention may be implemented using other analytical multi-criteria decision modelling methods such as aggregated indices randomization method (AIRM), analytic network process (ANP), multi-attribute global inference of quality (MAGIQ), weighted sum model (WSM), best worst method (BWM), and decision expert (DEX).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A pregnancy decision support system (10) for identifying potentially high-risk combinations of pregnancy-related symptoms, comprising:
a first input interface (12) for receiving a categorization of symptoms that a pregnant woman may report;
a model-building unit (20) adapted to build a model for identifying potentially high-risk combinations of pregnancy-related symptoms based on the received categorization of symptoms;
a second input interface (14) for receiving information about symptoms reported by the pregnant woman;
a controller (30) adapted to apply the reported symptoms to the model and thereby determine a proposed course of action; and
an output interface (40) for outputting the proposed course of action.

2. The system of claim 1, wherein the first input interface (12) is further for receiving a categorization of symptom attributes comprising one or more of duration, frequency, intensity and location.

3. The system of claim 1 or 2, wherein the first input interface (12) is for receiving different categorizations of symptom attributes for different phases of pregnancy.

4. The system of any one of claims 1 to 3, wherein the proposed course of action indicates whether the pregnant women should seek medical assistance and/or indicates a type of care package that should be implemented by a medical professional.

5. The system of any one of claims 1 to 4, wherein the model-building unit (20) is adapted to apply an analytic hierarchy process.

6. The system of claim 5, wherein the model-building unit is adapted to generate a pairwise comparison matrix of symptoms and calculate therefrom weights of each symptom.

7. The system of any one of claims 1 to 6, wherein the symptoms comprise a plurality selected from: headache; edema; visual disturbances; abdominal pain; pain in the stomach pit; pain between the shoulders; nausea; vomiting; loss of appetite; malaise; loss of fetal motion; finger tingling; urinary problems; hyper sensitivity; and hyperreflexia.

8. A computer implemented method for identifying potentially high-risk combinations of pregnancy-related symptoms, comprising:
receiving a categorization of symptoms that a pregnant woman may report;
building a model for identifying potentially high-risk combinations of pregnancy-related symptoms based on the received categorization of symptoms;
receiving information about symptoms reported by the pregnant woman;
applying the reported symptoms to the model and thereby determining a proposed course of action; and
outputting the proposed course of action.

9. The method of claim 8, comprising receiving the categorization of symptoms from multiple medical professionals as qualitative data, and the method comprises converting the qualitative data into quantitative data before building the model.

10. The method of claim 8 or 9, comprising:
building a first model based on received categorizations from multiple medical professionals and building a second model based on received categorization of symptoms from an individual medical professional; or
building a first model based on received categorizations from a first group of multiple medical professionals and building a second model based on received categorization of symptoms from a second group of multiple medical professionals.

11. The method of any one of claims 8 to 10, comprising receiving a categorization of symptoms derived from medical professional surveys or from a user interface allowing the categorizations to be applied to the symptoms.

12. The method of any one of claims 8 to 11, comprising receiving a categorization of symptom attributes comprising one or more of duration, frequency, intensity and location.

13. The method of any one of claims 8 to 12, comprising receiving different categorizations of symptom attributes for different phases of pregnancy.

14. The method of any one of claims 8 to 11, wherein the symptoms comprise a plurality selected from: headache; edema; visual disturbances; abdominal pain; pain in the stomach pit; pain between the shoulders; nausea; vomiting; loss of appetite; malaise; loss of fetal motion; finger tingling; urinary problems; hyper sensitivity; and hyperreflexia.

15. A computer program comprising computer program code which is adapted, when said program is run on a computer, to implement the method of any one of claims 8 to 14.
